# EUROPEAN PATENT APPLICATION

(11) **EP 2 286 768 A2**
(43) Date of publication of application: **23.02.2011**
(21) Application number: 09425383.8
(22) Date of filing: 30.09.2009
(51) Int. Cl.: A61F 2/32, A61F 2/34

(54) **Prosthetic models for arthroprosthetic hip operations**

(30) Priority: 17.08.2009 IT NO20090004
(71) Applicant: Zonca, Vincenzino, I-28060 Recetto (NO) (IT)
(72) Inventor: Zonca, Vincenzino, I-28060 Recetto (NO) (IT)

(57) **Abstract**

Acetabulum, plus the addition of a half-sphere portion to be screwed to it; a dissection must be made between the stem and the head so to make the setting of the half-sphere section into the acetabulum easier and - in the meantime - to choose the most suitable stem as for shape and size to be infibulated.

## Description

Making of half-sphere acetabula by adding a perfectly tallying portion of half-sphere, to be screwed to. The side adhering to the acetabulum will include a hollow which is due to stick to the stem head; the outer side will be roundish-shaped in order to make the stem mobility easier. The added part will be just a few (3-4) millimetres thick (as shown in images 1, 2, 3). Stem dissection is due to facilitate the addition and the fitting of half-sphere portions in different shapes and to use infibulating sections differing as for shape and size (see image 4).

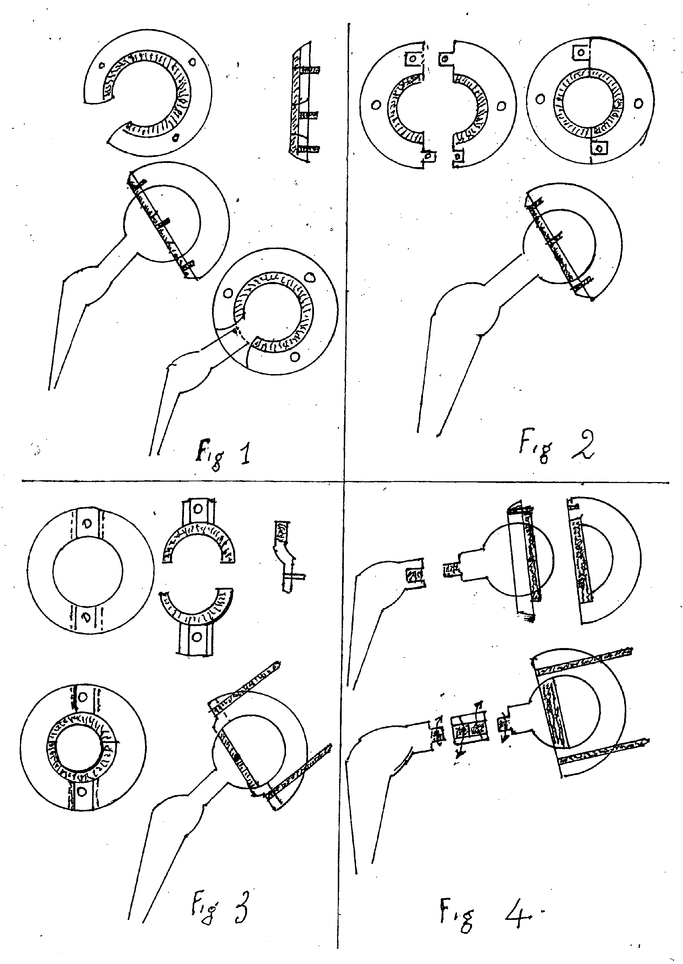

## Claims

1. The invention makes any post-operational displacement impossible.

2. The dissection of the stem makes it possible to choose among several stems differing as for shape and size, in accordance with the needs.

3. The invention makes moving (squatting, crossing legs, putting on socks and shoes, doing rehabilitation exercises) safer and easier.
